(19) Europäisches Patentamt
European Patent Office
Office européen des brevets

(11) Numéro de publication: **0 003 920**
**B1**

(12) # FASCICULE DE BREVET EUROPEEN

(21) Numéro de dépôt: **79400053.9**

(22) Date de dépôt: **26.01.79**

(51) Int. Cl.[3]: **C 07 D 495/04,**
A 61 K 31/44, //
(C 07 D 495/04, 333/00,
221/00)

(54) **Thiéno (3,2-c) et thiéno (2,3-c) pyridines, leur procédé de préparation et leur application en thérapeutique**

(30) Priorité: **17.02.78 FR 7804561**

(43) Date de publication de la demande:
**05.09.79 Bulletin 79/18**

(45) Mention de la délivrance du brevet:
**07.01.81 Bulletin 81/01**

(84) Etats contractants désignés:
**DE NL SE**

(56) Documents cités:
**US - A - 3 845 065**

(73) Titulaire: **OMNIUM FINANCIER AQUITAINE POUR L'HYGIENE ET LA SANTE - SANOFI**
**Tour Aquitaine Cedex No 4**
**F - 92080 Paris La Defense (FR)**

(72) Inventeur: **Frehel, Daniel**
**Résidence La Pléiade 28 Boulevard de la Marne**
**F - 31400 Toulouse (FR)**
**Maffrand, Jean-Pierre**
**10 Rue Jean Mermoz**
**F - 31300 Toulouse (FR)**

(74) Mandataire: **Lavoix, Jean, et al**
**c/o Cabinet Lavoix 2, Place D'Estienne D'Orves**
**F - 75441 Paris Cedex 09 (FR)**

**EP 0 003 920 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Thiéno [3,2-c] et thiéno [2,3-c] pyridines, leur procédé de préparation et leur application en thérapeutique

L'invention est relative à de nouvelles thiéno [3,2-c] et [2,3-c] pyridines, à un procédé pour les préparer et à leurs applications en thérapeutique. Les nouveaux dérivés suivant l'invention répondent à l'une ou l'autre des formules suivantes:

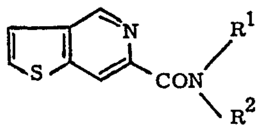

et

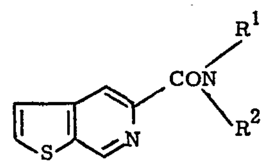

dans lesquelles $R^1$ et $R^2$ sont chacun, indépendamment l'un de l'autre, l'hydrogène; un groupe alcoyle en $C_1$ à $C_8$; un groupe alcényle en $C_2$ ou $C_3$; un groupe alcynyle en $C_2$ ou $C_3$; un groupe phényle ou phényl-alcoyle en $C_1$ ou $C_2$ éventuellement substitués sur le noyau phényle par un ou plusieurs atomes d'halogène ou groupes alcoyle de $C_1$ à $C_4$, alcoxy de $C_1$ à $C_4$, hydroxy ou trifluorométhyle; un groupe hétéroaralcoyle, notamment pyridyl-alcoyle inférieur dans lequel le groupe alcoyle inférieur contient 1 ou 2 atomes de carbone; ou un groupe de formule

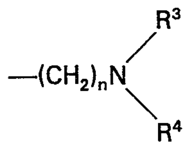

dans lequel n est 2 our 3 et $R^3$ et $R^4$ sont indépendamment l'un de l'autre un radical alcoyle en $C_1$ à $C_4$ ou bien forment, ensemble et avec l'atome d'azote auquel ils sont rattachés, une hétérocycle à 5 ou 6 chaînons, pouvant comporter un second hétéroatome; ou bien $R^1$ et $R^2$ forment, ensemble et avec l'atome d'azote auquel ils sont rattachés, un hétérocycle à 5 ou 6 chaînons pouvant comporter un second hétéroatome, choisi parmi l'oxygène, le soufre et l'azote, ce dernier pouvant porter un groupe alcoyle en $C_1$ à $C_4$, un groupe benzyle ou un groupe phényle éventuellement substitué par un ou plusieurs atomes d'halogène ou groupes alcoyle en $C_1$ à $C_4$, alcoxy en $C_1$ à $C_4$ ou trifluorométhyle.

L'invention comprend aussi les sels d'addition avec des acides minéraux ou organiques pharmaceutiquement acceptables de ces dérivés.

Lorsque $R^1$ et/ou $R^2$ représentent un groupe phényl-alcoyle, celui-ci est notamment un groupe benzyle ou phénéthyle; lorsqu'ils représentent un groupe pyridyl-alcoyle inférieur, celui-ci est notamment un groupe (pyridyl-3) méthyle ou un groupe (pyridyl-4) méthyle.

A titre d'état de la technique, on citera le brevet US 3 845 065 qui décrit des 4-oxo-4,5-dihydrothiéno [3,2-c] pyridines ayant une activité thérapeutique.

L'invention a également pour objet un procédé de préparation des composés de formule (I) ou (II) ci-dessus, caractérisé en ce que l'on fait réagir une amine de formule

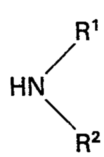

dans lequelle $R^1$ et $R^2$ ont les mêmes significations que précédemment avec un anhydride mixte de formule (III) ou (IV) dans lequel R est un groupe alcoyle en $C_1$—$C_3$

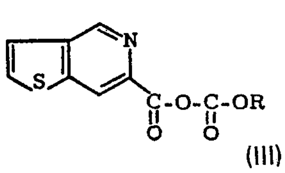

(III)

ou

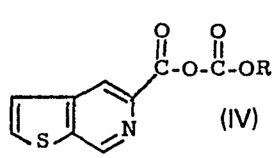

(IV)

obtenant ainsi respectivement les dérivés de formule (I) ou (II).

Les composés de départ (III) et (IV) sont eux-mêmes préparés par condensation, en présence de triéthylamine, de thiénopyridines de formule (V) ou (VI)

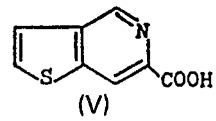

(V)

ou

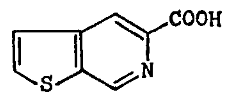

(VI)

avec un chloroformiate d'alcoyle de formule ClCOOR dans lequel R a la signification donnée précédemment.

Les deux réactions sont de préférence effectuées successivement dans le même récipient: les anhydrides mixtes (III) et (IV) sont d'abord préparés à des températures comprises entre −5 et +15°C au sein d'un solvant inerte, tel que le chloroforme ou le chlorure de méthylène; on ajoute ensuite, à la même température, l'amine

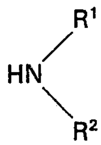

pure ou dissoute dans un solvant tel que le benzène, le toluène, le chloroforme ou le chlorure de méthylène, et on abandonne le mélange à température ambiante.

Les thiénopyridines (V) et (VI) utilisées comme matières premières peuvent être préparées par un procédé suivant lequel on fait réagir un composé de formule

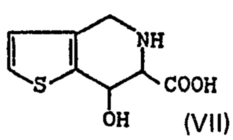

(VII)

ou

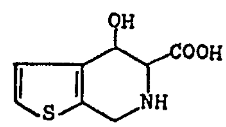

(VIII)

avec de l'acide nitreux, puis on déshydrate et élimine le groupe nitroso des composés obtenus par réaction avec un hydroxyde de métal alcalin et neutralisation subséquente.

Les composés de départ de formule (VII) ou (VIII) peuvent aux-mêmes être préparés par réaction d'un composé de formule

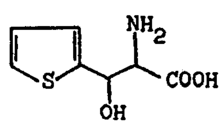

ou

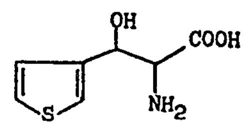

avec du formaldéhyde en solution aqueuse, en présence d'un acide fort.

Les exemples non limitatifs suivants sont donnés à titre d'illustration de l'invention.

Exemple 1

Méthylaminocarbonyl-6 thiéno [3,2-c] pyridine

Formule (I): $NR^1R^2 = NHCH_3$ — dérivé No 1

A une solution maintenue à 10°C, de 10 g (0,050 mole) de carboxy-6 thiéno [3,2-c] pyridine et de 5,6 g (0,057 mole) de triéthylamine, dans 500 cm³ de chloroforme sec, on ajoute lentement, sous agitation vigoureuse, 6,2 g (0,057 mole) de chloroformiate d'éthyle. L'introduction terminée, on poursuit l'agitation à température ambiante pendant 40 minutes, puis ajoute, goutte à goutte, une solution de 2 g (0,064 mole) de méthylamine dans 50 cm³ de benzène. On abandonne à température ambiante pendant 4 heures, évapore à sec et reprend le résidu par de l'éther. La phase éthérée est lavée avec une solution aqueuse saturée de bicarbonate de sodium, séchée sur sulfate de sodium et évaporée à sec.

Le résidu solide est recristallisé dans un mélange benzène-éther diisopropylique.

Cristaux rosâtres, F = 99°C, rendement: 79%.

Exemple 2

β-diméthylaminoéthylaminocarbonyl-6 thiéno [3,2-c] pyridine

Formule (I): $NR^1R^2 = NH(CH_2)_2N(CH_3)_2$ — dérivé No 2

A une solution maintenue à 10°C de 10 g (0,056 mole) de carboxy-6 thiéno [3,2-c] pyridine et de 5,6 g (0,057 mole) de triéthylamine dans 300 cm³ de chloroforme sec, on ajoute lentement, sous agitation vigoureuse, 6,2 g (0,057 mole) de chloroformiate d'éthyle. L'introduction terminée, on

poursuit à température ambiante pendant 40 minutes, puis ajoute, goutte à goutte, 5,4 g (0,061 mole) de β-diméthylaminoéthylamine. On abandonne à température ambiante pendant 3 heures 30, évapore à sec et reprend le résidu par de l'acide chlorhydrique N. La phase aqueuse acide est lavée avec de l'éther puis alcalinisée par de la soude 6N, et extraite avec du chlorure de méthylène. Les extraits chlorométhyléniques sont séchés sur sulfate de sodium sec et évaporés à sec. Le résidu huileux est purifié par l'intermédiaire de son dichlorhydrate.
Cristaux beiges, F = 170°C (isopropanol-méthanol), rendement: 75%

Exemple 3
(p-chlorophényl-4 pipérazinyl-1) carbonyl-5 thiéno [2,3-c] pyridine

Formule (II): $NR_1R_2$ = 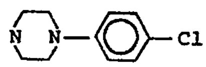 dérivé No 3

A une solution, maintenue à 10°C, de 12 g (0,067 mole) de carboxy-5 thiéno [2,3-c] pyridine et de 6,9 g (0,068 mole) de triéthylamine dans 250 $cm^3$ de chloroforme sec, on ajoute lentement, sous agitation vigoureuse, 7,3 g (0,068 mole) de chloroformiate d'éthyle. On poursuit l'agitation à température ambiante pendant 50 minutes et ajoute, goutte à goutte, 13,2 g (0,067 mole) de p-chlorophényl pipérazine dissous dans 50 $cm^3$ de chloroforme. On abandonne à température ambiante pendant 5 heures, évapore à sec et reprend le résidu par du chlorure de méthylène. La phase chlorométhylénique est lavée avec une solution aqueuse de bicarbonate de sodium saturée, séchée sur sulfate de sodium sec et évaporée à sec. Les cristaux obtenus sont purifiés par chromatographie sur colonne de silice (éluant acétate d'éthyle).
Cristaux blancs, F = 170°C (isopropanol-éther diisopropylique) rendement: 41%.

Exemple 4
Ethylaminocarbonyl-6 thiéno [3,2-c] pyridine

Formule (I): $NR^1R^2 = NHC_2H_5$ — dérivé No 4

Préparée selon le mode opératoire décrit à l'exemple 1 à partir de carboxy-6 thiéno [3,2-c] pyridine et d'éthylamine.
Cristaux beiges, F = 110° C (éther diisopropylique), rendement 87%.

Exemple 5
Isopropylaminocarbonyl-5 thiéno [2,3-c] pyridine

Formule (II): $NR^1R^2 = NHC_3H_7$ — dérivé No 5

Préparée selon le mode opératoire décrit à l'exemple 1 à partir de carboxy-5 thiéno [2,3-c] pyridine et d'isopropylamine.
Cristaux marron clair, F = 102°C (éther diisopropylique), rendement: 80%.

Exemple 6
n-butylaminocarbonyl-6 thiéno [3,2-c] pyridine

Formule (I): $NR^1R^2 = NHC_4H_9$ — dérivé No 6

Préparée selon le mode opératoire décrit à l'exemple 2 à partir de carboxy-6 thiéno [3,2-c] pyridine et de n-butylamine.
Chlorhydrate, cristaux jaune orangé, F = 104°C (acétonitrile), rendement:

Exemple 7
Octylaminocarbonyl-6 thiéno [3,2-c] pyridine

Formule (I): $NR^1R^2 = NHC_8H_{17}$ — dérivé No 7

Préparée selon le mode opératoire décrit à l'exemple 1 à partir de carboxy-6 thiéno [3,2-c] pyridine et d'octylamine.
Cristaux blancs, F = 63°C (éther diisopropylique), rendement: 54%.

Exemple 8
Diméthylaminocarbonyl-6 thiéno [3,2-c] pyridine

Formule (I): $NR^1R^2 = N(CH_3)_2$ — dérivé No 8

Préparée selon le mode opératoire décrit à l'exemple 1 à partir de carboxy-6 thiéno [3,2-c]

pyridine et de diméthylamine.
Cristaux blancs, F = 93°C (éther diisopropylique), rendement: 55%.

Exemple 9
Diéthylaminocarbonyl-6 thiéno [3,2-c] pyridine

Formule (I): $NR^1R^2 = N(C_2H_5)_2$ — dérivé No 9

Préparée selon le mode opératoire décrit à l'exemple 1 à partir de carboxy-6 thiéno [3,2-c] pyridine et de diméthylamine.
Cristaux beiges, F = 119°C (éther diisopropylique), rendement: 80%.

Exemple 10
(pyrrolidinyl-1)carbonyl-6 thiéno [3,2-c] pyridine

Formule (I): $NR^1R^2 =$ 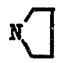 — dérivé No 10

Préparée selon le mode opératoire décrit à l'exemple 1 à partir de carboxy-6 thiéno [3,2-c] pyridine et de pyrrolidine.
Cristaux blanchâtres, F = 105°C (éther diisopropylique), renement: 52%.

Exemple 11
Pipéridinocarbonyl-6 thiéno [3,2-c] pyridine

Formule (I) = $NR^1R^2 =$ 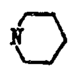 — dérivé No 11

Préparée selon le mode opératoire décrit à l'exemple 1 à partir de carboxy-6 thiéno [3,2-c] pyridine et de pipéridine.
Poudre marron clair, F = 145°C (éther diisopropylique), rendemment: 96%.

Exemple 12
Morpholinocarbonyl-6 thiéno [3,2-c] pyridine

Formule (I): $NR^1R^2 =$ 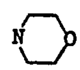 — dérivé No 12

Préparée selon le mode opératoire décrit à l'exemple 1 à partir de carboxy-6-thiéno [3,2-c] pyridine et de morpholine.
Cristaux blancs, F = 136°C (benzène-éther diisopropylique), rendement: 79%.

Exemple 13
Benzylaminocarbonyl-5 thiéno [2,3-c] pyridine

Formule (II): $NR^1R^2 = NHCH_2C_6H_5$ — dérivé No 13

Préparée selon le mode opératoire décrit à l'exemple 1 à partir de carboxy-5 thiéno [2,3-c] pyridine et de benzylamine.
Cristaux beiges, F = 113°C (isopropanol), rendement: 75%.

Exemple 14
o-chlorobenzylaminocarbonyl-6 thiéno [3,2-c] pyridine

Formule (I): $NR^1R^2 = NHCH_2$ 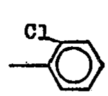 — dérivé No 14

Préparée selon le mode opératoire décrit à l'exemple 1 à partir de carboxy-6 thiéno [3,2-c] pyridine et d'o-chlorobenzylamine.
Poudre beige, F = 169°C (méthanol), rendement: 58%.

Exemple 15

Phénéthylaminocarbonyl-6 thiéno [3,2-c] pyridine

Formule (I) $NR^1R^2$ $NHCH_2CH_2C_6H_5$ dérivé No 15

Préparée selon le mode opératoire décrit à l'exemple 1 à partir de carboxy-6 thiéno [3,2-c] pyridine et de phénéthylamine

Cristaux beiges F = 127°C (isopropanol éther diisopropylique) rendement 66%

Exemple 16

Allylaminocarbonyl-6 thiéno [3,2-c] pyridine

Formule (I). $NR^1R^2$ $NHCH_2-CH=CH_2$ dérivé No 16

Préparée selon le mode opératoire décrit à l'exemple 1 à partir de carboxy-6 thiéno [3,2-c] pyridine et d'allylamine.

Oxalate, cristaux blancs, F = 131°C (acétate d'éthyle), rendement 54%

Exemple 17

Propargylaminocarbonyl-6 thiéno [3,2-c] pyridine

Formule (I) $NR^1R^2$ $NHCH_2C \equiv CH$ dérivé No 17

Préparée selon le mode opératoire décrit à l'exemple 1 à partir de carboxy-6 thiéno [3,2-c] pyridine et de propargylamine

Cristaux rosâtres F = 134°C (isopropanol-éther diisopropylique) rendement 60%

Exemple 18

$\beta$-diéthylaminoéthylaminocarbonyl-6 thiéno [3,2-c] pyridine

Formule (I): $NR^1R^2 = NH(CH_2)_2N(C_2H_5)_2$ — dérivé No 18

Préparée selon le mode opératoire décrit à l'exemple 2 à partir de carboxy-6 thiéno [3,2-c] pyridine et de $\beta$-diéthylaminoéthylamine.

Dichlorhydrate, cristaux beiges, F = 145°C (isopropanolméthanol), rendement: 81%.

Exemple 19

$\beta$-morpholinoéthylaminocarbonyl-6 thiéno [3,2-c] pyridine

Formule (I): $NR^1R^2 = NH(CH_2)_2$ 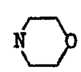 — dérivé No 19

Préparée selon le mode opératoire décrit à l'exemple 2 à partir de carboxy-6 thiéno [3,2-c] pyridine et de N-(aminoéthyl-2)-morpholine.

Cristaux blancs, F = 104°C (isopropanol-éther diisopropylique), rendement: 71%.

Exemple 20

$\gamma$-diméthylaminopropylaminocarbonyl-6 thiéno [3,2-c] pyridine

Formule (I): $NR^1R^2 = NH(CH_2)_3N(CH_3)_2$ — dérivé No 20

Préparée selon le mode opératoire décrit à l'exemple 2 à partir de carboxy-6 thiéno [2,3-c] pyridine et de $\gamma$-diméthylaminopropylamine

Dichlorhydrate cristaux beiges, F = 146°C (éthanol) rendement: 77%.

Exemple 21

(pyridyl-4-méthyl)aminocarbonyl 5-thiéno [2,3-c] pyridine

Formule (II): $NR^1R^2 = NHCH_2$ 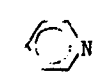 – dérivé No 21

Préparée selon le mode opératoire décrit à l'exemple 3 à partire de carboxy-5 thiéno [2,3-c] pyridine et de l'aminométhyl-4 pyridine

Cristaux marron clair F = 167°C (isopropanol-éther diisopropylique), rendement 78%

Exemple 22

(pyridyl-4 méthyl)aminocarbonyl-6 thiéno [3,2-c] pyridine

Formule (I): $NR^1R^2$ = NHCH$_2$ 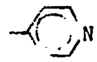 — dérivé No 22

Préparée selon le mode opératoire décrit à l'exemple 3 à partir de carboxy-6 thiéno [3,2-c] pyridine et de l'aminométhyl-4 pyridine.
Cristaux orangés, F = 146°C (acétate d'éthyle), rendement: 98%.

Exemple 23

(pyridyl-3 méthyl)aminocarbonyl-5 thiéno [2,3-c] pyridine

Formule (II): $NR^1R^2$ = NHCH$_2$ 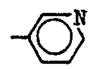 — dérivé No 23

Préparée selon le mode opératoire décrit à l'exemple 3 à partir de carboxy-5 thiéno [2,3-c] pyridine et de l'aminométhyl-3 pyridine.
Cristaux beiges, F = 143°C (isopropanol-éther diisopropylique), rendement: 73%.

Exemple 24

(pyridyl-3 méthyl)aminocarbonyl-6 thiéno [3,2-c] pyridine

Formule (I): $NR^1R^2$ = NHCH$_2$ 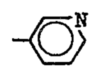 dérivé No 24

Préparée selon le mode opératoire décrit à l'exemple 3 à partir de carboxy-6 thiéno [3,2-c] pyridine et de l'aminométhyl-3 pyridine.
Cristaux beiges, F = 137°C (acétate d'éthyle), rendement: 55%.

Exemple 25

(trifluorométhyl-3 phényl)aminocarbonyl-6 thiéno [3,2-c] pyridine

Formule (I): $NR^1R^2$ = NH 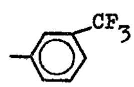 — dérivé No 25

Préparée selon le mode opératoire décrit à l'exemple 1 à partir de carboxy-6 thiéno [3,2-c] pyridine et de m-trifluorométhylaniline.
Cristaux blancs, F = 151°C (isopropanol-éther diisopropylique), rendement: 62%.

Exemple 26

(p-tolyl-4 pipérazinyl-1) carbonyl-6 thiéno [3,2-c] pyridine

Formule (I): $NR^1R^2$ = 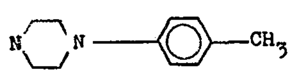 dérivé No 26

Préparée selon le mode opératoire décrit à l'exemple 3 à partir de carboxy-6 thiéno [3,2-c] pyridine et de p-tolyl-1 pipérazine.
Cristaux beiges, F = 150°C (isopropanol-éther diisopropylique), rendement: 82%.

Exemple 27

(o-chlorophényl-4 pipérazinyl-1) carbonyl-6 thiéno [3,2-c] pyridine

Formule (I): $NR^1R^2$ = 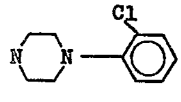 — dérivé No 27

Préparée selon le mode opératoire décrit à l'exemple 3 à partir de carboxy-6 thiéno [3,2-c] pyridine et de l'o-chlorophényl-1 pipérazine.
Cristaux beiges, F = 140°C (isopropanol-éther diisopropylique), rendement: 52%.

Exemple 28
(m-chlorophényl-4 pipérazinyl-1) carbonyl-6 thiéno [3,2-c] pyridine

Formule (I): $NR^1R^2$ = 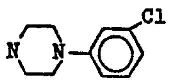 — dérivé No 28

Préparée selon le mode opératoire décrit à l'exemple 3 à partir de carboxy-6 thiéno [3,2-c] pyridine et de la m-chlorophényl-1 pipérazine.
Cristaux blancs, F = 157°C (acétate d'éthyle), rendement: 52%.

Exemple 29
(p-méthoxyphényl-4 pipérazinyl-1) carbonyl-6 thiéno [3,2-c] pyridine

Formule (I): $NR^1R^2$ = 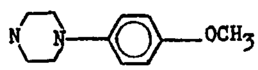 — dérivé No 29

Préparée selon le mode opératoire décrit à l'exemple 3 à partir de carboxy-6 thiéno [3,2-c] pyridine et de la p-méthoxyphényl-1 pipérazine.
Cristaux blancs, F = 152°C (acétate d'éthyle-éther diisopropylique), rendement: 72%.

Exemple 30
(o-méthoxyphényl-4 pipérazinyl-1) carbonyl-5 thiéno [2,3-c] pyridine

Formule (II): $NR^1R^2$ = 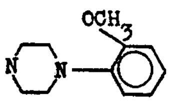 — dérivé No 30

Préparée selon le mode opératoire décrit à l'exemple 3 à partir de carboxy-5 thiéno [2,3-c] pyridine et de l'o-méthoxyphényl-1 pipérazine.
Cristaux beiges, F = 171°C (isopropanol), rendement: 62%.

Exemple 31
carbamoyl-6 thiéno [3,2-c] pyridine

Formule (I): $NR^1R^2 = NH_2$ — dérivé No 31

Préparée selon le mode opératoire décrit à l'exemple 1 à partir de carboxy-6 thiéno [3,2-c] pyridine et d'ammoniac.
Cristaux blancs, F = 172°C (acétonitrile), rendement: 68%.

Exemple 32
Carbamoyl-5 thiéno [2,3-c] pyridine

Formule (II): $NR^1R^2 = NH_2$ — dérivé No 32

Préparée selon le mode opératoire décrit à l'exemple 1 à partir de carboxy-5 thiéno [2,3-c] pyridine et d'ammoniac.
Cristaux blancs, F = 200°C (acétonitrile), rendement: 76%.

Exemple 33
phénéthylaminocarbonyl-5 thiéno [2,3-c] pyridine

Formule (II): $NR^1R^2 = NHCH_2CH_2C_6H_5$ — dérivé No 33

Préparée selon le mode opératoire décrit à l'exemple 1 à partir de carbonyl-5 thiéno [2,3-c] pyridine et de phénéthylamine.
Cristaux beiges, F = 130°C (isopropanol-éther diisopropylique), rendement: 79%.

Exemple 34
(benzyl-4 pipérazinyl-1) carbonyl-6 thiéno [3,2-c] pyridine

Formule (I): $NR^1R^2 =$ 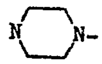 $CH_2C_6H_5$ — dérivé No 34

Préparée selon le mode opératoire décrit à l'exemple 3 à partir de carboxy-6 thiéno [3,2-c] pyridine et de benzyl-1 pipérazine.
Dichlorhydrate, cristaux blancs, F = 187°C (isopropanol-éthanol), rendement: 49%.

Exemple 35
(diméthoxy-3,4 phénéthyl) aminocarbonyl-6 thiéno [3,2-c] pyridine

Formule (I): $NR^1R^2 = NHCH_2CH_2$ 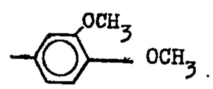 — dérivé No 35

Préparée selon le mode opératoire décrit à l'exemple 1 à partir de carboxy-6 thiéno [3,2-c] pyridine et de (diméthoxy-3,4-phénéthyl)amine.
Cristaux blancs, F = 125°C (isopropanol-éther diisopropylique) rendement: 77%.

Exemple 36
(diméthoxy-3,4 phénéthyl)aminocarbonyl-5- thiéno [2,3-c] pyridine

Formule (II): $NR^1R^2 = NHCH_2CH_2$ 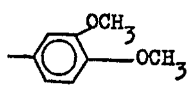 — dérivé No 36

Préparée selon le mode opératoire décrit à l'exemple 1 à partir de carboxy-5 thiéno [2,3-c] pyridine et de (diméthoxy-3,4 phénéthyl)-amine.
Cristaux blancs, F = 125°C (isopropanol-éther diisopropylique), rendement: 73%.

Exemple 37
(méthyl-4 pipérazinyl-1) carbonyl-6 thiéno [3,2-c] pyridine

Formule (I): $NR^1R^2 =$ 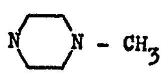 — dérivé No 37

Préparée selon le mode opératoire décrit dans l'exemple 1 à partir de carboxy-6 thiéno [3,2-c] pyridine et de méthyl-1 pipérazine.
Maléate, poudre marron, F = 168°C (isopropanol), rendement: 83%.

Les résultats pharmacologiques et toxicologiques qui sont rapportés ci-dessous ont mis en évidence les propriétés des dérivés de l'invention tant sur le plan de la toxicité et de la tolérance que sur le plan de leurs activités, notamment sédative, anti-convulsivante et anti-inflammatoire.

L'invention a donc encore pour objet une composition thérapeutique présentant en particulier des activités sédative, anti-convulsivante et anti-inflammatoire, caractérisée en ce qu'elle contient, à titre de principe actif, un dérivé de formule (I) ou (II) ou un sel d'addition avec un acide pharmaceutiquement acceptable de celui-ci, et un véhicule thérapeutiquement administrable.

## I — Etude toxicologique

Les composés de l'invention bénéficient d'une excellente tolérance et d'une faible toxicité.

En ce qui concerne la toxicité aigüe, la $DL_{50}$/24 H/kg de poids corporel, déterminée chez la souris selon la méthode de Miller et Tainter pour la voie orale est supérieure à 400 mg pour tous les dérivés.

Selon la même méthode, la $DL_{50}$/24 H/kg de poids corporel déterminée par la voie intraveineuse est, par exemple, de 154 mg pour le dérivé No 1, de 89 mg pour le dérivé No 2, de 184 mg pour le dérivé No 10, de 130 mg pour le dérivé No 11, de 350 mg pour le dérivé No 12, de 65 mg pour le dérivé No 18, de 90 mg pour le dérivé No 22, de 96 mg pour le dérivé No 24 et de 105 mg pour le dérivé No 31.

En outre, les essais effectués sur la toxicité aigüe, chronique, sub-chronique et retardée, chez différentes espèces animales n'ont pas permis de mettre en évidence une quelconque réaction locale ou générale, perturbation ou anomalie dans les examens biochimiques, microscopiques ou macroscopiques effectués durant cette expérimentation.

## II — Etude pharmacologique

1) Action sédative

A) Etude du comportement

Cette étude a été effectuée selon la méthode de Samuel IRWIN (Ph. D. Animal and Clinical Pharmacology Technics in drug evaluation). Les dérivés de l'invention sont administrés par la voie orale à des souris à la dose de 100 mg/kg. Les animaux traités sont observés pendant les 4 heures qui suivent l'administration du médicament. Leur comportement est étudié et on effectue en outre la mesure des différents paramètres physiologiques (température, vitesse cardiaque et respiratoire). On observe chez les animaux traités une nette diminution de l'activité motrice et du tonus musculaire ainsi qu'une baisse de l'état d'alerte et des réactions au bruit et à l'environnement.

B) Action vis-à-vis des hypnotiques

Les composés de l'invention potentialisent de façon très nette l'action des hypnotiques. En effet, administrés à différents lots de souris par la voie orale, à la dose de 100 mg/kg, trente minutes avant l'injection intrapéritonéale d'une dose infra-hypnotique de pentobarbital sodique, ils produisent par rapport aux animaux témoins non traités, une nette potentialisation de l'action du barbiturique.

En effet, le nombre de souris endormies, le temps moyen d'endormissement et la durée du sommeil sont nettement accrus dans les lots traités. Les résultats des composés les plus actifs sont rassemblés dans le Tableau I suivant.

Tableau I

| Traitement | Pourcentage d'animaux endormis | Temps moyen d'endormissement | Durée moyenne du sommeil |
|---|---|---|---|
| 0(lot témoin) | 0 | 0 | 0 |
| Dérivé 1 | 70 | 8 mn 30 s | 1 h 30 mn |
| Dérivé 5 | 80 | 9 mn 15 s | 1 h 45 mn |
| Dérivé 6 | 80 | 8 mn 40 s | 1 h 48 mn |
| Dérivé 10 | 90 | 8 mn 25 s | 1 h 35 mn |
| Dérivé 15 | 90 | 8 mn 10 s | 1 h 50 mn |
| Dérivé 16 | 70 | 7 mn 50 s | 1 h 42 mn |
| Dérivé 18 | 80 | 9 mn 45 s | 1 h 38 mn |
| Dérivé 22 | 70 | 9 mn 20 s | 1 h 45 mn |
| Dérivé 23 | 80 | 7 mn 55 s | 1 h 50 mn |
| Dérivé 25 | 90 | 8 mn 10 s | 1 h 38 mn |
| Dérivé 26 | 90 | 8 mn 50 s | 1 h 35 mn |
| Dérivé 28 | 80 | 7 mn 45 s | 1 h 40 mn |
| Dérivé 29 | 90 | 8 mn 15 s | 1 h 47 mn |

2) Action anti-convulsivante

Cette action a été étudiée vis-à-vis des électrochocs. L'application chez le rat d'une stimulation électrique supérieure au seuil électro-convulsivant provoque des convulsions expérimentales. On compare alors chez les animaux témoins et chez les animaux traités, à la fois la présence et la durée de chacune des phases convulsives et l'intensité de la crise dans son ensemble.

On établit des séries de 10 rats par produit à tester et on administre par la voie orale, à chaque animal, 100 mg/kg de ce produit.

De chaque côté de la base de la queue de chaque souris, une électrode est mise en place et 30 minutes après le traitement, l'animal placé dans une enceinte de verre reçoit pendant 50 millisecondes un courant sinusoïdal de 50 périodes/secondes de 120 volts.

Le passage du courant déclenche une crise convulsive dont chacune des phases (tonique, clonique, relachement musculaire) est chronométrée. L'intensité de la crise est alors notée de 0 à 4 selon la présence de chacune des phases et leur durée. Les produits de l'invention sont testés comparativement au phénobarbital qui possède une action anti-convulsivante marquée (intensité de la crise = 2), alors que chez les animaux témoins non traités c'est l'intensité maxima de 4 qui est obtenue.

On détermine ainsi que tous les dérivés de l'invention produisent une protection importante vis-à-vis de l'électrochoc puisque les valeurs moyennes de l'intensité des crises à l'intérieur de chaque lot sont de 2,5 pour le dérivé No 1, 3 pour le dérivé No 4, 2,5 pour le dérivé No 5, 2,5 pour le dérivé No 9, 3 pour le dérivé No 13, 2,5 pour le dérivé No 19, 2,5 pour le dérivé No 25, 2,5 pour le dérivé No 30 et 2,5 pour le dérivé No 31.

3) Action anti-inflammatoire

A) Méthode de l'oedème localisé provoqué par la carragénine

Une solution de carragénine (0,1 ml), à 1%, est injectée dans les fléchisseurs métatarsiens de la patte postérieure droite du rat au temps 0. Les animaux du lot traité reçoivent en outre, par la voie orale, 100 mg par kg du dérivé à tester respectivement 1 heure avant, en même temps que l'injection de l'agent phlogogène, puis 1 heure et 2 heures et demi après. Les mesures qui sont effectuées à l'aide du micromètre de ROCH au temps 0, 1 heure, 2 heures, 3 heures et 5 heures après l'administration de la carragénine permettent de déterminer, en fonction du temps, le pourcentage d'activité anti-inflammatoire, par comparaison avec le lot témoin.

Les résultats concernant certains des dérivés de formule I ou II sont consignés dans le Tableau II suivant:

Tableau II

| Dérivé No | Pourcentage d'activité anti-inflammatoire 1ère heure | 2ème heure | 3ème heure | 5ème heure |
|---|---|---|---|---|
| 1 | 35 | 42 | 46 | 47 |
| 4 | 41 | 46 | 50 | 58 |
| 5 | 43 | 49 | 54 | 56 |
| 10 | 37 | 42 | 46 | 51 |
| 13 | 34 | 40 | 45 | 51 |
| 15 | 38 | 45 | 49 | 53 |
| 18 | 41 | 46 | 50 | 55 |
| 21 | 40 | 48 | 51 | 54 |
| 22 | 41 | 47 | 51 | 55 |
| 25 | 39 | 46 | 49 | 52 |
| 27 | 41 | 49 | 52 | 54 |
| 30 | 38 | 45 | 49 | 53 |
| 32 | 34 | 41 | 45 | 47 |

B) Méthode de l'oedème généralisé à l'ovalbumine

Une injection intrapéritonéale simultanée de 1 ml d'ovalbumine et de 0,5 ml d'une solution aqueuse de bleu Evans à 1% est effectuée sur le rat. D'autre part, on administre per os aux animaux du lot traité 100 mg/kg du dérivé à tester une heure avant et en même temps que l'ovalbumine. L'intensité du phénomène ainsi provoqué est notée par un chiffre allant de 1 à 5 suivant la progression du syndrome inflammatoire. On détermine ainsi la moyenne de l'intensité oedémateuse et le pourcentage de diminution de la réaction oedémateuse par rapport au témoin, en fonction du temps.

Les pourcentages d'activité anti-inflammatoire obtenus à la 2ème heure et la 3ème heure après l'injection d'ovalbumine sont consignés dans le Tableau III suivant pour certains dérivés de l'invention.

Tableau III

| Dérivé No | Pourcentage d'activité anti-inflammatoire 2ème heure | 3ème heure |
|---|---|---|
| 1 | 48 | 56 |
| 4 | 47 | 56 |
| 5 | 52 | 60 |
| 10 | 47 | 55 |
| 13 | 50 | 56 |
| 15 | 53 | 60 |
| 18 | 49 | 58 |
| 21 | 45 | 52 |
| 22 | 51 | 58 |
| 25 | 51 | 59 |
| 27 | 47 | 56 |
| 30 | 48 | 56 |
| 32 | 52 | 60 |

Les résultats de ces études mettent en évidence la faible toxicité et la bonne tolérance ainsi que les intéressantes propriétés sédative, anti-convulsivante et anti-inflammatoire des dérivés de l'invention qui les rendent très utiles en médecine humaine et vétérinaire.

La composition de l'invention peut être présentée, pour l'administration orale, sous forme de comprimés, comprimés dragéifiés, capsules, gouttes et sirop. Elle peut aussi être présentée, pour l'administration rectale, sous forme de suppositoires et pour l'administration parentérale, sous forme de soluté injectable.

Chaque dose unitaire contient avantageusement de 0,050 g à 0,500 g de principe actif, les doses administrables journellement pouvant varier de 0,050 g à 1,50 g de principe actif selon l'âge du patient et l'affection traitée.

On donnera ci-après, à titre d'exemples non limitatifs, quelques formulations pharmaceutiques de la composition de l'invention.

1 — Comprimés
Dérivé No 1 0,250 g
Excipient: amidon de maïs, stéarate de magnésium, acide stéarique.

2 — Comprimés dragéifiés
Dérivé No 5 0,150 g
Excipient: stéarate de magnésium, amidon de maïs, gomme arabique, gomme laque, sucre, glucose, cire blanche, cire de carnauba, lactose, huile de ricin, alcool, laque tartrazine aluminium.

3 — Capsules
Dérivé No 13 0,100 g
Excipient: stéarate de magnésium, amidon de maïs, lactose.

4 — Suppositoires
Dérivé No 24 0,150 g
Triglycérides semi-synthétiques q.s.p. 1 suppositoire.

5— Soluté injectable
Dérivé No 31 0,100 g
Solvant isotonique q.s.p. 5 ml

Par leur action sédative et anti-convulsivante, les dérivés de l'invention réduisent les troubles du caractère et du comportement et facilitent les rapports avec l'entourage grâce à un meilleur équilibre psychique. Ils sont indiqués chez l'enfant et l'adulte dans les cas d'agressivité, irritabilité, instabilité, excitation et agitation psychomotrice ainsi que toutes les manifestations d'excitabilité.

Par leur action anti-inflammatoire, administrés en traitement court ou prolongé, ils interviennent efficacement dans la réaction inflammatoire pour contrôler l'oedème, l'hypersécrétion et l'exsudation au cours des différents stades de l'inflammation. Ils sont indiqués dans les rhumatismes inflammatoires chroniques, les rhumatismes dégénératifs, les affections ab-articulaires, la goutte aigüe, en chirurgie réparatrice et plastique post-opératoire, en traumatologie, en otorhino-laryngologie.

## Revendications

1. Composés de formule

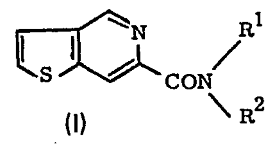

(I) ou

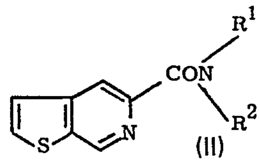

(II)

dans lesquelles $R^1$ et $R^2$ sont chacun indépendamment l'un de l'autre, l'hydrogène; un groupe alcoyle en $C_1$ à $C_8$; un groupe alcényle en $C_2$ ou $C_3$; un groupe alcynyle en $C_2$ ou $C_3$; un groupe phényle ou phénylalcoyle en $C_1$ ou $C_2$ éventuellement substitués sur le noyau phényle par un ou plusieurs atomes d'halogène ou groupes alcoyle de $C_1$ à $C_4$, alcoxy de $C_1$ à $C_4$, hydroxy ou trifluorométhyle; un groupe pyridyl-alcoyle inférieur dans lequel le groupe alcoyle inférieur contient 1 ou 2 atomes de carbone; ou un groupe de formule

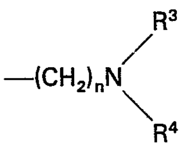

dans lequel n est 2 ou 3 et $R^3$ et $R^4$ sont indépendamment l'un de l'autre un radical alcoyle en $C_1$ à $C_4$ ou bien forment, ensemble et avec l'atome d'azote auquel ils sont rattachés, un hétérocycle à 5 ou 6 chaînons, pouvant comporter un second hétéroatome; ou bien $R^1$ et $R^2$ forment, ensemble et avec l'atome d'azote auquel ils sont rattachés, un hétérocycle à 5 ou 6 chaînons pouvant comporter un second hétéroatome, choisi parmi l'oxygène, le soufre et l'azote, ce dernier pouvant porter un groupe alcoyle en $C_1$ à $C_4$, un groupe benzyle ou un groupe phényle éventuellement substitué par un ou plusieurs atomes d'halogène ou groupes alcoyle en $C_1$ à $C_4$, alcoxy en $C_1$ à $C_4$ ou trifluorométhyle; et leurs sels d'addition avec des acides pharmaceutiquement acceptables.

2. Procédé de préparation des composés suivant la revendication 1, caractérisé en ce que l'on fait réagir une amine de formule

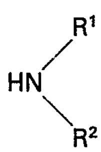

dans laquelle $R^1$ et $R^2$ ont les mêmes significations que ci-dessus avec un anhydride mixte de formule (III) ou (IV) dans lequel R est un groupe alcoyle en $C_1$—$C_3$

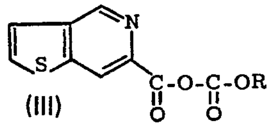

(III) ou

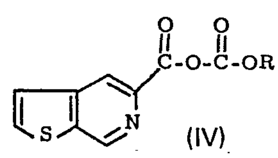

(IV)

obtenant ainsi respectivement les dérivés de formule (I) ou (II).

3. Procédé suivant la revendication 2, caractérisé en ce qu'on condense, en présence de triéthylamine, des thiénopyridines de formule (V) ou (VI)

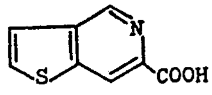 ou 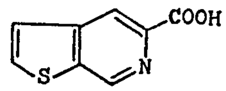

(V) (VI)

avec un chloroformiate d'alcoyle de formule ClCOOR dans lequel R a la signification donnée précédemment, obtenant ainsi les composés de formule (III) ou (IV).

4. Procédé suivant la revendication 2, caractérisé en ce qu'on effectue la réaction dans un solvant inerte à une température de −5 à +15°C.

5. Procédé suivant la revendication 3, caractérisé en ce qu'on effectue la réaction dans un solvant inerte à une température de −5 à +15°C.

6 Composition thérapeutique présentant en particulier des activités sédative, anti-convulsivante et anti-inflammatoire, caractérisée en ce qu'elle contient, à titre de principe actif, un dérivé de formule (I) ou (II) suivant la revendication 1 ou un sel d'addition avec un acide pharmaceutiquement acceptable de celui-ci, et un véhicule thérapeutiquement administrable.

7. Composition thérapeutique suivant la revendication 6, caractérisé en ce qu'elle est présentée sous forme de doses unitaires.

8. Composition thérapeutique suivant la revendication 7, caractérisé en ce que chaque dose unitaire contient de 0,050 à 0,500 g de principe actif.

**Claims**

1. Compounds having the formula:

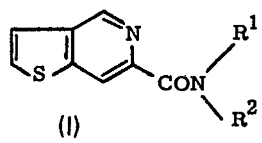 and 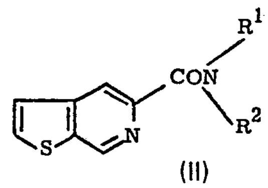

(I) (II)

in which $R^1$ and $R^2$ are independently: hydrogen; a $C_1$—$C_8$ alkyl group; a $C_2$—$C_3$ alkenyl group; a $C_2$—$C_3$ alkynyl group; a phenyl or phenyl-($C_1$—$C_2$) alkyl group optionally substituted on the phenyl nucleus with one or more halogen atoms of $C_1$—$C_4$ alkyl, $C_1$—$C_4$ alkoxy, hydroxy or trifluoromethyl groups; a pyridyl-lower alkyl group in which the lower alkyl radical contains 1 or 2 carbon atoms; or a group having the formula:

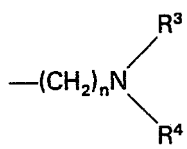

in which *n* is 2 or 3, and $R^3$ and $R^4$ are independently a $C_1$—$C_4$ alkyl radical or, together with the nitrogen atom to which they are attached, form a 5- or 6-membered heterocyclic which may carry a second heteroatom selected from oxygen, sulfur and nitrogen; or, together with the nitrogen atom to which they are attached, $R^1$ and $R^2$ form a 5- or 6-membered heterocycle which may carry a second heteroatom selected from oxygen, sulfur and nitrogen, which nitrogen may carry a $C_1$—$C_4$ alkyl group, a benzyl radical or a phenyl radical optionally substituted with one or more halogen atoms or $C_1$—$C_4$ alkyl, $C_1$—$C_4$ alkoxy or trifluoromethyl groups; and their pharmaceutically acceptable acid addition salts.

2. Process for the preparation of compounds as claimed in claim 1, comprising reacting an amine having the formula:

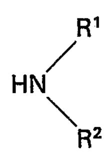

in which $R^1$ and $R^2$ are as claimed in claim 1, with a mixed anhydride having the formula (III) or (IV) in which R is a $C_{1-3}$ alkyl group:

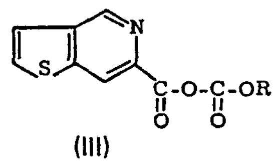

(III) or

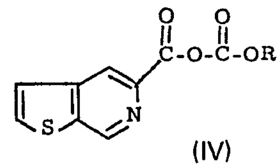

(IV)

to give a derivative of the formula (I) or (II), respectively.

3. Process as claimed in claim 2, wherein thienopyridines of the formula (V) or (VI)

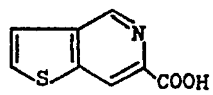

(V) or

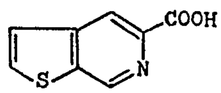

(VI)

are condensed, in the presence of triethylamine, with an alkyl chloroformate having the formula ClCOOR in which R has the meaning given in claim 2, to give the compounds of the formula (III) or (IV), respectively.

4. Process as claimed in claim 2, wherein the reaction is effected within an inert solvent at a temperature between −5°C and +15°C.

5. Process as claimed in claim 3, wherein the reaction is effected within an inert solvent, at a temperature between −5°C and +15°C.

6. Therapeutic composition having, in particular, sedative, anti-convulsant and anti-inflammatory activites, comprising, as active ingredient, a derivative of the formula (I) or (II) as claimed in claim 1 or a pharmaceutically acceptable acid addition salt thereof, together with a therapeutically administrable carrier.

7. Therapeutic composition as claimed in claim 6, in unit dosage form.

8. Therapeutic composition as claimed in claim 7, wherein each unit dose contains from 0.050 g to 0.500 g active ingredient.

**Patentansprüche**

1. Verbindungen der Formeln

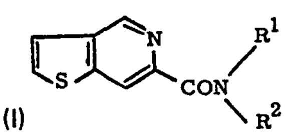

(I) oder

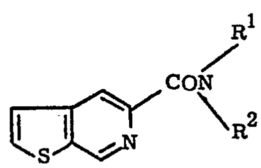

(II)

worin $R^1$ und $R^2$ unabhängig voneinander jeweils Wasserstoff; $C_{1-8}$ Alkyl; $C_{2-3}$ Alkenyl; $C_{2-3}$ Alkinyl; Phenyl oder Phenyl-$C_{1-2}$ alkyl, gegebenenfalls am Phenylring ein oder mehrfach durch Halogen, $C_{1-4}$ Alkyl, $C_{1-4}$ Alkoxy, Hydroxy oder Trifluormethyl substituiert; Pyridyl-$C_{1-2}$ alkyl oder eine Gruppe der Formel

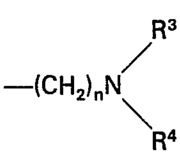

bedeuten, wobei n 2 oder 3 ist und $R^3$ und $R^4$ unabhängig voneinander jeweils $C_{1-4}$-Alkyl bedeuten oder zusammen mit dem Stickstoffatom, und das sie gebunden sind, eine 5- oder 6-gliedrige heterocyclische Gruppe bilden, die als zweites Heteroatom ein Sauerstoff-, Schwefel- oder Stickstoffatom tragen kann; oder $R^1$ und $R^2$ zusammen mit dem Stickstoffatom, an das sie gebunden sind, eine 5- oder 6-gliedrige heterocyclische Gruppe bilden, die als zweites Heteroatom ein Sauerstoff-, Schwefel- oder Stickstoffatom tragen kann, welch letzteres durch $C_{1-4}$ Alkyl, Benzyl oder Phenyl substituiert sein kann, welches seinerseits gegebenenfalls durch mindestens ein Halogenatom oder eine $C_{1-4}$ Alkyl-, $C_{1-4}$ Alkoxy- oder Trifluormethylgruppe substituiert ist, und deren Säureadditionssalzen mit pharmazeutisch verwendbaren Säuren.

2. Verfahren zur Herstellung von der Verbindungen nach Anspruch 1 dadurch gekennzeichnet, dass man ein Amin der allgemeinen Formel

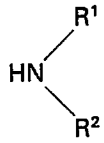

worin $R^1$ und $R^2$ die obige Bedeutung haben, mit einem gemischten Anhydrid der allgemeinen Formel

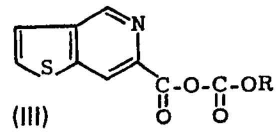

(III)

oder

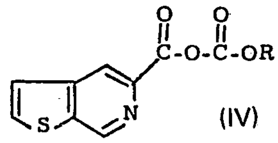

(IV)

worin R $C_{1-3}$-Alkyl darstellt, umzetzt, wobei Verbindungen der Formeln (I) oder (II) erhalten wurden.

3. Verfahren nach Anspruch 2, dadurch gekennzeichnet, dass man Thienopyridinen der Formeln

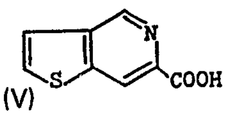

(V)

oder

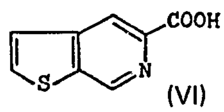

(VI)

mit einem Alkylchlorformiat der Formel ClCOOR kondensiert, worin R die in Anspruch 1 angegebene Bedeutung hat, in Anwesenheit von Triäthylamin, wobei Verbindungen der Formeln (III) oder (IV) erhalten wurden.

4. Verfahren nach Anspruch 2, dadurch gekennzeichnet, dass man die Reaktion in einem inerten Lösungsmittel bei einer Temperatur von −5 bis +15°C durchführt.

5. Verfahren nach Anspruch 3, dadurch gekennzeichnet, dass man die Reaktion in einem inerten Lösungsmittel bei einer Temperatur von −5 bis +15°C durchführt.

6. Therapeutisches Mittel mit insbesondere sedativen, antikonvulsiven und entzündungshemmenden Wirksamkeiten, dadurch gekennzeichnet dass es eine Verbindung der Formel (I) oder (II) nach Anspruch 1 oder einen Säureadditionssalz mit einem pharmazeutisch verwendbaren Säure als Wirkstoff, und einen therapeutisch verabreichbaren Träger, enthält.

7. Therapeutisches Mittel nach Anspruch 6, dadurch gekennzeichnet dass es in Dosiseinheiten formuliert ist.

8. Therapeutisches Mittel nach Anspruch 7, dadurch gekennzeichnet dass jede Dosiseinheit 0,050 bis 0,500 g Wirkstoff enthält.